(19) Europäisches Patentamt
European Patent Office
Office européen des brevets

(11) **EP 1 121 865 B1**

(12) **EUROPEAN PATENT SPECIFICATION**

(45) Date of publication and mention
of the grant of the patent:
**16.03.2005 Bulletin 2005/11**

(51) Int Cl.⁷: **A23L 1/30**, A61K 31/23

(21) Application number: **00309676.5**

(22) Date of filing: **01.11.2000**

(54) **A medical composition for the treatment and/or prevention of malnutrition**

Medizinische Zusammensetzung zur Behandlung und/oder zur Verhinderung von Unterernährung

Composition medicale pour le traitement et / ou la prevention de la malnutrition

| | |
|---|---|
| (84) Designated Contracting States:<br>**AT BE CH CY DE DK ES FI FR GB GR IE IT LI LU MC NL PT SE TR** | (74) Representative: **Lees, Kate Jane et al**<br>**Roystons,**<br>**Tower Building,**<br>**Water Street**<br>**Merseyside, Liverpool L3 1BA (GB)** |
| (30) Priority: **04.11.1999 GB 9925997** | |
| (43) Date of publication of application:<br>**08.08.2001 Bulletin 2001/32** | (56) References cited:<br>**EP-A- 0 229 938**    **EP-A- 0 266 323**<br>**GB-A- 840 091**    **US-A- 4 781 937**<br>**US-A- 5 284 674** |
| (73) Proprietor: **Vitaflo Limited**<br>**Liverpool L3 4BL (GB)** | |
| (72) Inventors:<br>• **O'Donnell, Maura**<br>**Hightown, Merseyside L38 3RT (US)**<br>• **Partington, Thomas**<br>**Liverpool, Merseyside L3 4EE (US)** | • **DATABASE WPI Section Ch, Week 200020 Derwent Publications Ltd., London, GB; Class D13, AN 2000-231243 XP002168917 & JP 2000 050841 A (AMANO JITSUGYO KK), 22 February 2000 (2000-02-22)** |

EP 1 121 865 B1

**Description**

The present invention relates to a medical composition for the improved treatment and/or prevention of malnutrition.

It is widely documented that a large percentage of patients are malnourished upon admission to hospital and that most continue to lose weight during their stay in hospital. (For Example, see McWhirler and Pennington; "Incidence and recognition of malnutrition in hospital"; BMJ 1994; 308; 945-948). It has been estimated that food intakes are less than 75% of that recommended and that 30 to 50% of hospital food is wasted. This is clearly undesirable in relation to the recovery of the patient and additionally, is a severe economic burden to hospitals where financial resources are limited, with an estimated quarter of million pounds being wasted on food per year in a large hospital. Additionally, the inability to provide patients with their optimal nutritional intake adversely effects their prognosis and may lead to a lengthened hospital stay.

Up to now there have been two main approaches for increasing energy and protein intake in undernourished patients, the use of low energy density liquid supplements and increasing the nutrient density of food. However, the former must be consumed in high volumes due to its low energy which tends to result in it displacing the patient's normal food intake leading to no overall increase in energy intake. Additionally, patients tend to become tired of the same flavours and textures of the liquid supplements which may result in patients not taking the supplement as prescribed.

Increasing the nutrient density of food by fortification is of major benefit since people tend to consume a constant weight of food rather than a constant quantity of energy. Accordingly, if a person consumes a set volume of food, then the more energy rich the food the greater will be their energy intake. Fortification also helps create normal eating patterns. A number of nutritional products have been used to fortify foods which have met with varying degrees of success. Glucose polymers contribute 4kcal per g but have limited use because of their inherent sweetness, rendering them unsuitable for savoury food or any foods at high doses. Traditional protein supplements are difficult to disperse and disguise in foods and drinks and fat emulsions and fat/carbohydrate mixes have taste problems and are expensive. Currently, the highest level of energy intake that can be achieved using a fortifier is 5.14 kcal per g.

EP-A-0 266 323 relates to a granulate for producing a food supplement in the form of a tablet comprising an oil-powder mixture and a water-soluble carrier in combination with a solid pulverent filler and a binder. GB 840 091 describes a food supplement containing a high concentration of edible protein. US 5 284 674 and EP 0 229 938 concern creamer-thickeners having up to around 25% by weight fat. US 478937 describes a rehydratable dehydrated food product having 10-35% by fat.

It is an object of the present invention to provide a medical composition for the improved treatment and/or prevention of malnutrition that aims to overcome the abovementioned drawbacks.

Accordingly, the present invention provides a medical composition for fortifying foods and drinks for the treatment and/or prevention of malnutrition characterised in that the composition is in powdered form and consists of nutrients in the following percentages by weight of the total composition; at least 40% by weight fat; 15-30% by wight carbohydrate; 3-30% by weight protein; and optionally, 10-25% by weight fibre and minor amounts of vitamins and/or minerals.

The composition preferably contains at least 50% fat, more preferably still at least 60%, especially at least 70%, most especially at least 75% . The fat source may be, for example, palm oil or soya oil.

Preferably, the composition of the present invention provides an energy intake of at least 5kcal per gram, more preferably at least 6 kcal per gram, especially at least 7kcal per g.

The composition is suitable for fortifying a wide range of foods and drinks without adversely effecting the taste, texture or volume of the food or drink. The composition may be added to, for example, porridge, soup, scrambled egg, creamed or mashed potato, pureed food, gravy, custard, chocolate sauce, milk puddings, milk jelly, tea/coffee, hot chocolate and mousse.

It is to be appreciated that the dosage of the composition to be included in a patient's meal should be determined by a clinician or dietician and is dependent upon age, body weight and clinical condition of the patient.

Other nutrients may be included in the composition of the present invention, such as minerals and vitamins. Preferably, the composition contains 4 to 15% protein. Any suitable source of powdered protein may be used, such as sodium casienate, skimmed milk powder or gluten.

The carbohydrate source may be, for example, lactose, glucose, multi-dextrin or a starch derivative.

The composition may also include a specified amount of fibre for administering to patients that require an additional fibre intake. Preferably, such a composition includes 10 to 25 % fibre, more preferably 15 to 20%. The fibre may be included as soluble or insoluble fibre. Preferably, approximately equal amounts of soluble and insoluble fibre are included in the composition.

It is preferable to provide the composition of the present invention in satchets holding a specified weight to provide a predetermined amount of energy from the satchet, such as 100kcal per satchet. Additionally, the composition may be provided in bulk containers having a scoop therein for measuring a specified amount of composition, for example one level scoop of the composition provides 100kcal.

A preferred composition of the present invention contains nutrients in the following percentages by weight:-

| Fat | 77 ± 5 |
|---|---|
| Carbohydrate | 17 ± 5 |
| Protein | 4.6 ± 5 |

to provide an energy intake of at least 7kcal per gram.

Alternatively, the amount of fat in the composition may be reduced to allow an increased amount of protein to be included thereby providing a composition that is both an energy and protein food/drink enricher.

A preferred composition for providing energy and protein contains nutrients in the following percentages by weight:-

| Fat | 56.2 ± 5 |
|---|---|
| Carbohydrate | 26.8 ± 5 |
| Protein | 13.5 ± 5 |

to provide an energy intake of at least 6kcal per gram and a protein intake of at least 0.12g per gram.

Alternatively, the amount of fat and/or protein may be reduced to allow for a quantity of fibre to be included in the composition thereby providing a composition that has a high fibre content as well as providing an energy and/ or protein food/drink enricher.

A preferred composition for providing energy, protein and fibre contains nutrients in the following percentages by weight:-

| Fat | 43.4 ± 5 |
|---|---|
| Carbohydrate | 27.6 ± 5 |
| Protein | 8.4 ± 5 |
| Fibre | 17.2 ± 5 |

to provide an energy intake of at least 5kcal per gram, a protein intake of at least 0.075g per gram and a fibre intake of at least 0.15g per gram.

Minor amounts of minerals and/or vitamins are preferably included in the composition, for example in amounts representing the recommended daily intake of said minerals and/or vitamins.

The present invention will now be illustrated with reference to the following Examples in which Example 1 illustrates the content of a medical composition according to one embodiment of the present invention and demonstrates its efficiency in increasing the energy intake of patients who are, or at risk of, losing weight during long stay residential care, Example 2 illustrates the content of a medical composition according to an alternative embodiment of the present invention and compares its efficiency at enriching the energy and protein content of foods to a conventional food fortifier consisting of glucose polymer and skimmed milk powder in a group of end stage renal failure patients and Example 3 illustrates the content of a composition according to yet another embodiment of the present invention, and with reference to the accompanying drawings in which:-

Figure 1 is a graph illustrating the mean energy intake, served and consumed, for baseline (no added fortifier) and intervention trials (QuickCal™ added) (P<0.00 1);

Figure 2 is a graph illustrating mean energy of unfortified food in baseline and intervention trials (served P=0.142; consumed P=0.565);

Figure 3 is a graph illustrating the mean daily energy intakes, served and consumed, for patients in low, moderate and high nutrient risk groups;

Figure 4 is a graph illustrating the mean difference in daily energy intake between baseline and intervention studies; and

Figure 5 is a graph illustrating the percentage waste for low, moderate and high nutrient risk groups, in baseline and intervention trials.

**Example 1**

One of the preferred compositions according to the present invention is a powder known as QuickCal™, which is a blend of fat, protein and carbohydrate of high energy density (7.8 kcal/g). This powder has been specifically designed to fortify many types of foods and drinks with energy with the minimum effect on taste, volume and texture.

The typical composition of QuickCal™ is given in Table 1 below:-

Table 1

|  | **Per 100g** | **Per 13g** |
|---|---|---|
| Energy kj (kcal) | 3216 (780) | 424 (100) |
| Protein (g) | 4.6 | 0.6 |
| Carbohydrate (g) | 17.0 | 2.2 |
| Fat (g) | 77.0 | 10.0 |

The fat contributes 88.85% of the energy in the composition and is in powdered form. The inclusion of such a high percentage of fat in powder form is an important factor in the uniqueness of the present invention wherein a high energy fortifier may be incorporated into many types of food and drink without adversely effecting taste, texture or volume. The fat source in QuickCal™ is palm oil and the fatty acid profile is given in Table 2 below:-

Table 2

| **Type of Fat** | **%** |
|---|---|
| Saturated | 54.0 |
| Mono unsaturated | 42.0 |
| Polyunsaturated | 4.0 |

The composition also includes some protein which is derived from sodium casienate but other protein sources could be used, such as gluten. A typical protein composition profile in mg per 100g QuickCal™ is as follows:-

| L-Tryptophan | 58 | L-Valine | 301 |
|---|---|---|---|
| L-Threonine | 175 | L-Arginine | 167 |
| L-Isoleucine | 255 | L-Histidine | 125 |
| L-Leucine | 413 | L-Alanine | 129 |
| L-Lysine | 334 | L-Aspartic acid | 296 |
| L-Methionine | 125 | L-Glutamic acid | 949 |
| L-Cystine | 16 | Glycine | 20 |
| L-Phenylalanine | 225 | L-Proline | 88 |
| L-Tyrosine | 238 | L-Serine | 259 |

Carbohydrate contributes 8.72% of the energy in QuickCal™ and the source is lactose. However, it is to be appreciated that a number of other sources may be used, such as galactose, multi-dextrose or a starch derivative.

Table 3 below provides details of the energy source in QuickCal™.

Table 3

| **Source** | **Derivation of kcal per 100g** | **Derivation of KJ per 100g** |
|---|---|---|
| Protein | 18.4 | 78.2 |
| Carbohydrate | 68.0 | 289.0 |
| Fat | 693.0 | 2849.0 |
| **Total Energy** | **779.4** | **3216.2** |

The electrolyte and mineral content of the composition is given in Table 4.

Table 4

| Mineral | | Per 100g | Per 13g |
|---|---|---|---|
| Sodium | (mg) | 100 | 13 |
| | (mmol) | 4.3 | 0.6 |
| Potassium | (mg) | <10 | <13 |
| | (mmol) | <0.25 | <0.03 |
| Chloride | (mg) | 0.0 | 0.0 |
| | (mmol) | 0.0 | 0.0 |
| Calcium | (mg) | <10 | <1.3 |
| Phosphorus | (mg) | <20 | <2.6 |

The shelf life of the composition was also investigated since it is desirable to produce a composition with a long shelf life. Samples of packs of the composition were stored in a warehouse at ambient temperature (min recorded temperature = 19°C, max = 24°C) arid were tested for Microbiology at time 0 and every six months, and analyzed for Peroxide value, free fatty acids, moisture and sensory evaluation after 1 month, 2 months and then every 3 months. There was no significant change in any parameter after 12 months and accordingly, a 12 month shelf life is recommended for the composition. This is a further benefit provided by the present invention since conventional fortifiers, such as butter and cream, have only a short shelf life.

A clinical trial was also conducted to determine the efficiency of the composition QuickCal™ to increase the energy intake of patients who are, or at risk of, losing weight during long-stay residential care.

32 subjects (15 male and 17 female) were recruited from a psychogeriatric ward, all of whom had dementia. They were aged between 58 and 97 with the mean average age being 76 years old. Nutritional Assessment was carried out to determine the prevalence of undernutrition by use of a questionnaire and by measuring the patients' weight. Questions asked included details of length of stay in that ward, age, weight and height on admission, last recorded weight, weight change, visual appearance (BMI >25, 19-25, <19), appetite, ability to eat and drink, diet, swallowing ability, mental state, stress factors and Waterlow Score. The patients were weighted before and after the study and the nutritional status of each patient was determined from the questionnaire and measured weights. The prevalence of undernutrition was also established.

A four-day food frequency questionnaire was performed to determine the foods to be fortified and a seven day weighed food intake was carried out to find the baseline daily energy intake. Patients were placed into high, moderate and low nutritional risk groups depending on their nutritional status and deficit in mean daily energy intake. Another 7 day weighed food intake was performed with the patients receiving fortified food according to their fortification group.

Foods included on the menu that had the potential to be fortified were id entified as porridge, scrambled egg, creamed potato, puree, gravy, custard, chocolate sauce, milk puddings, milk jelly, tea/coffee and hot chocolate/Horlicks™.

A baseline weighted food intake was carried out in the first week of the menu cycle from Sunday to Saturday inclusive for all meals: breakfast, lunch, supper, snacks (drinks and snacks eaten between meals) and extras (food and drinks brought in by relatives). A weighed food inventory record using digital weighing scales (Soehnle) was performed. The nurses recorded the patients' snack intake (the drinks and snacks served and consumed) on a table provided. This provided baseline data of nutrient intake (mean daily energy intake in kJ/d) and waste (food served minus food consumed in kJ/d). The patients' weights were used to determine their basal metabolic rates using Schofield (1985) equations:

$$\text{Males: BMR - 0.049 x weight + 2.459}$$

$$\text{Females: BMR = 0.038 x weight + 2.755}$$

The BMR was modified for each patient according to their activity levels (active patients - 1.5 x BMR; inactive patients - 1.4 x BMR) to estimate their daily energy requirements. The mean daily deficit in energy for each patient was calculated by finding the difference between their energy intake in the baseline trial and their estimated requirements.

The patients were divided into the three groups according to their nutritional risk determined from the nutritional assessment questionnaire and the deficits in mean daily energy intake in the baseline trial. The low risk group patients (7) were either on a reducing diet or had energy intakes greater than their requirements. The moderate risk group (13 patients) had deficits in energy intake of less than 2000kJ per day. The criteria for inclusion in the high-risk group (5 patients) were one or more of high activity (agitated/wanderer), very low weight, puree diet and more than 2000kJ per day deficit in energy intake.

The target level of fortification was set at 836kJ or 200kcal per day per person. This relatively low target was set due to the patients' intakes, requirements and deficits being quite low. Another reason is because it was not feasible to exclude fortifying the low risk group's food due to the current organisation of preparation of food in the kitchen. Quickcal™ was added to the food and was found to mix easily into hot and cold foods, for example porridge, milk, puddings, custard, mashed potato and mashed banana.

The intervention weighted food intake was performed five weeks later during the first week of the menu cycle (the same week as in the baseline weighted food intake). In this week patients received food fortified according to their nutritional risk group. The low and moderate risk groups received food fortified in the hospital kitchen by the kitchen staff. The fortifier was mixed into creamed potato and custard at lunch and creamed potato at supper. These patients received a maximum of 1254kJ or 300kcal of fortifier per day. Patients in the high risk group received food fortified in the hospital kitchen as described above as well as an additional 836kJ or 200kcal of fortifier added to their food (for example porridge, coffee, puree, mashed banana) on the ward by the nurses. The nurses recorded the addition and consumption of the additional fortifier (each of four scoops) on a table provided for each of the patients in the high risk group. The mean daily intake of nutrients and the waste were calculated.

Of the 32 patients recruited for this study, 31 completed the baseline weighted food intake (one patient died), 28 started the intervention/fortified weighted food intake (3 patients died between the two study weeks) and 27 completed this week (one patient died). Of these 27, two patients had to be excluded due to illnesses they had during the intervention week. In total 25 patients completed both the baseline and intervention weeks.

All data were entered into an Excel database created using data from McCance and Widdowson's "The composition of Food" (by A.A. Paul and D.A.T. Southgate; 4th revised edition, 1978). This was used to calculate the mean daily intake of nutrients and energy, as well as the mean daily waste. Summary data were then entered into SPSS and Student's T-tests of matched pairs were carried out.

## Results

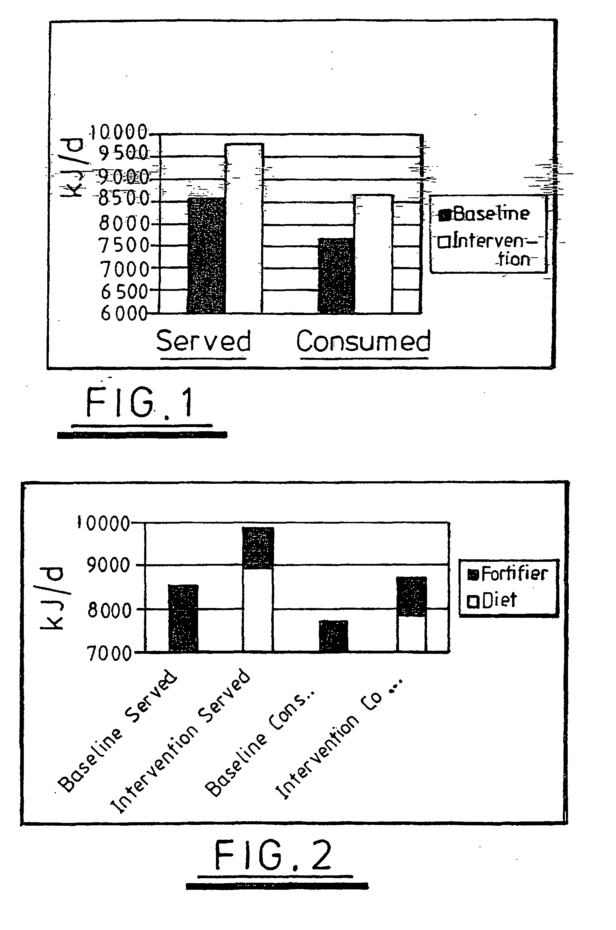

In the baseline trial patients were served a daily mean of 8577kJ ± 282kJ and in the intervention trail 9805kJ ± 350kJ - a significant difference of 1228kJ (P <0.001) which is 12.5% more food. In the intervention trial patients had a mean daily energy intake 12.9% higher than in the baseline trial (P <0.001), an increase of 996kJ (CI:449kJ to 1542kJ) per patient per day (from 7709kJ ± 311kJ in the baseline trail to 8705kJ ± 332kJ in the intervention trial). Figure 1 of the accompanying drawings illustrates the mean energy intake, served and consumed, for baseline and intervention trials (P < 0.001).

There was no statistically significant difference (P = 0.142) in the amount of food served in the baseline trial (8577kJ ± 282kJ) compared with the food served, excluding the fortifier, in the intervention trial (8896kJ ± 343kJ). Likewise, there was no difference (P = 0.565) in the amount of food without fortifier consumed in the baseline (7709kJ ± 322kJ) and the intervention trials (7836kJ ± 318kJ). Therefore, the increase in mean daily energy intake seen in the intervention trial was due to the fortifier alone, as illustrated in Figure 2.

Patients in the high nutritional risk group were served the least food in the baseline trial and consumed least. In the intervention trial they were served the most food and also consumed the greatest amount of food.

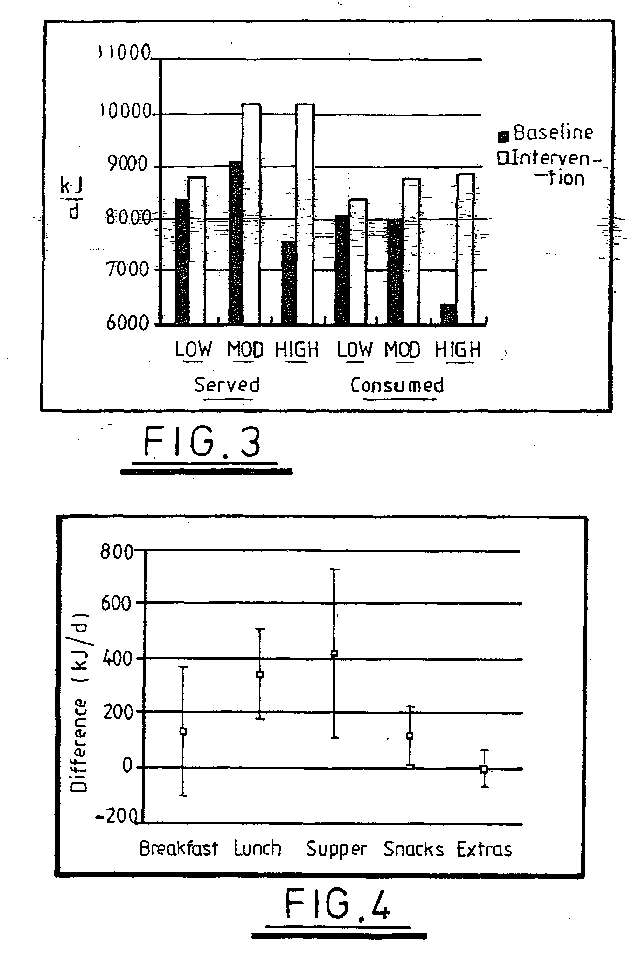

72% of patients increased their energy intake in the intervention trial. In all fortification groups an increase in mean daily energy served and consumed was seen in the intervention trial (P <0.05). The high nutritional risk group increased their energy intake the most in the intervention trial by 38% (2455kJ) (served: 7578kJ ± 690kJ in the baseline trial to 1019kJ ± 1204kJ in the intervention trial, a mean difference of 2617kJ consumed 6416kJ ± 425kJ in the baseline trial to 8871kJ ± 835kJ in the intervention trial, a mean difference of 2455kJ). All patients in the high nutritional risk group increased their energy intake significantly (P = 0.011). The moderate nutritional risk group as a whole increased their energy intake significantly by 10% (P = 0.03) although 4 patients out of 13 consumed less food in the intervention trial. The low nutritional risk group did not significantly increase their energy intake (P = 0.404). Three patients in this group ate less in the intervention trial. Figure 3 of the accompanying drawings demonstrates the mean daily intakes, served and consumed, per fortification group.

Statistically significant increases in mean daily energy intake were seen at lunch (P = 0.000), supper (P = 0.01) and snacks (P = 0.031). The greatest increase of 422.3kJ, or 17.6%, per patient per day was seen in supper although the confidence interval was wide (CI: 113kJ to 731.6kJ). Lunch showed a significantly large increase of 342.8kJ, that is 17.2%, with a much narrower confidence interval (CI: 178.4kJ to 507.3kJ). There was a small increase in the snacks of 120.7kJ (CI: 11.7kJ to 229.8kJ), as illustrated in Figure 4 of the accompanying drawings. No significant increase in mean energy intake was seen in breakfast or extras.

The difference in mean energy intake on days one and seven of the baseline and intervention trials was not statistically significant, as shown in Table 5.

Table 5

|  | Day 1 | Day 7 |
|---|---|---|
| Baseline | 7351kJ | 8592kJ |
| Intervention | 7491kJ | 19150kJ |
| Mean difference | 141kJ | 558kJ |
| Confidence Interval | 942kJ to 1223kJ | 676kJ to 1792kJ |
| P | 0.791 | 0.36 |

When analysing the intake of food by its composition there were statistically significant increases in intakes of protein and fat (P = 0.000). Intakes of protein and fat increased by 13.3% and 25% respectively in the intervention trial. There was no significant increase in the intakes of carbohydrate and fibre.

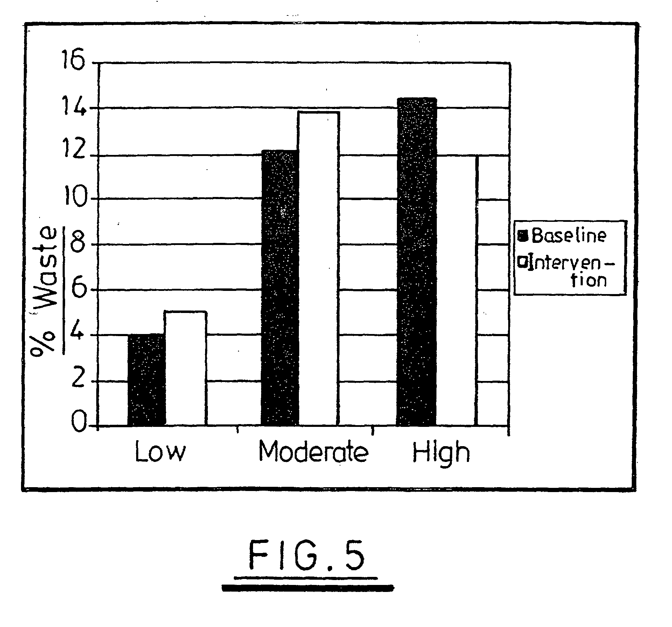

There was no overall increase in the percentage of waste in the intervention trial of 1.3%; the percentage waste in the baseline trial was 11.3% and in the intervention trial it was 12.64% (P = 0.031). In the low and moderate nutritional risk groups there was an increase in the percentage of waste in the intervention trial of 1.1% and 1.8% respectively, but in the high nutritional risk group the percentage waste was less in the intervention trial than in the baseline trial by 2.5% (14.5% in the baseline trial and 12% in the intervention trial), as shown in Figure 5.

909 kJ of fortifier were served in the intervention trial and 869 kJ was consumed. Only 4% of the fortifier was wasted.

The addition of the composition known as QuickCal™ in food and drink enabled patients in the high nutritional risk group to receive the most food in contrast to receiving the least food in the baseline trial. The mean daily energy intake increased by 13%, which is by 996kJ per patient per day. The increase in energy intake was seen in 84% of patients with the greatest increase of 38% seen in the high nutritional risk group. As there was no difference in the amount of food consumed in the intervention trial, this increase in energy intake may be attributed to the fortifier alone.

No fafigue was seen in this fortification trial of 7 days (i.e the energy intake increase was maintained). This may be due to the relatively short period of time in question not allowing for a fatigue effect to become apparent. Another factor may be that the fortifier was incorporated into normal hospital food without increasing the volume of food served to the patients, effectively hiding it. The target increase in energy intake was not large (although significant over a longer period of time) which may have prevented the patients' appetites being adversely affected. Another consideration is that the patients liked the fortified food. As the fortifier is mainly fat it made the fortified foods more creamy and appetising. Many of the patients studied have spent a considerable time (often years) in hospital and may have become bored of the menu that repeats every three weeks. It is possible that the fortifier enhanced the flavour and improved the consistency of the food.

Accordingly, it is clear that the composition QuickCal™ may be used to significantly increase the energy intake of psychogeriatric patients, without displacing food using a fortification approach. This increase is greatest in patients most at risk nutritionally.

## Example 2

Another powdered composition according to the present invention is known as ProCal™ and is a protein food enricher as well as an energy enricher. The composition provides 100kcal plus 2g protein per 15g and is made up of hydrogenated palm oil, skimmed milk powder, lactose and sodium casienate.

The typical composition of ProCal™ is given in Table 6 below:-

Table 6

|  | Per 100g | Per 15g |
|---|---|---|
| Energy kj (kcal) | 2788 (677) | 418 (100) |
| Protein (g) | 13.5 | 2 |
| Carbohydrate (g) | 26.8 | 4.0 |
| Fat (g) | 56.2 | 8.4 |

The protein is derived from the sodium casienate and skimmed milk powder. The typical protein composition profile in mg per 100g is as follows:-

| L-Tryptophan | 210 | L-Valine | 1069 |
|---|---|---|---|
| L-Threonine | 712 | L-Arginine | 583 |
| L-Isoleucine | 793 | L-Histidine | 437 |
| L-Leucine | 1458 | L-Alanine | 891 |
| L-Lysine | 1198 | L-Aspartic acid | 1830 |
| L-Methionine | 421 | L-Glutamic acid | 2592 |
| L-Cystine | 146 | Glycine | 486 |
| L-Phenylalanine | 794 | L-Proline | 939 |
| L-Tyrosine | 664 | L-Serine | 939 |

Table 7 below provides details of the energy source in ProCal™.

Table 7

| Source | Derivation of kcal per 100g | Derivation of kj per 100g |
|---|---|---|
| Protein | 54 | 229.5 |
| Carbohydrate | 107.2 | 455.6 |
| Fat | 505.8 | 2079.4 |
| Total Energy | 667 | 2764.5 |

16.07% of the energy is derived from carbohydrate, whereas fat contributes 75.83% of the energy of Pro-Cal™. The fatty acid profile is given in Table 8 below:-

Table 8

| | % |
|---|---|
| Saturated | 26.0 |
| Monounsaturated | 65.0 |
| Polyunsaturated | 9.0 |

The electrolyte and mineral and mineral content of Pro-Cal™ is given in Table 9 below:-

Table 9

| Mineral | | Per 100g | Per 13g |
|---|---|---|---|
| Sodium | (mg) | 250 | 37.5 |
| | (mmol) | 10.75 | 1.61 |
| Potassium | (mg) | 450 | 67.5 |
| | (mmol) | 11.25 | 1.69 |
| Chloride | (mg) | 295 | 44.2 |
| | (mmol) | 8.26 | 1.2 |
| Calcium | (mg) | 360 | 54 |
| Phosphorus | (mg) | 275 | 41.2 |
| Magnesium | (mg) | 40 | 6 |

As with QuickCal™, investigations into the shelf life of the composition established that there was no significant change in the composition after twelve months.

A clinical trial was conducted to compare the efficiency of ProCal™ to a conventional food fortification approach (consisting of glucose polymer and skimmed milk powder) in a group of end stage renal failure patients. Protein Calorie malnutrition is a frequent problem in patients with end stage renal failure, either in predialysis stage or after hemodialysis or peritoneal dialysis has been started. Malnutrition is mild to moderate in approximately 33% of maintenance dialysis patients and severe in approximately 6% to 8%.

20 patients were recruited (12 female, 8 male) from a Renal ward. Nutritional assessment was carried out to determine the Nutritional Risk Score of the patient. A patient data sheet was designed to elicit information regarding patients' nutritional status including weight (kg), height (m), BMI, mid-upper arm circumference (MUAC) and age. A brief clinical assessment of each patient including previous weight and dietary habits was documented. Patient information was reassessed on a weekly basis, until patients were discharged from the trail.

Patients were also visited on a regular basis by the researcher, to monitor any adverse symptoms such as nausea, vomiting, abdominal pain or diarrhoea. Patients were reassessed on a weekly basis until they were discharged from the trial. Patients were randomly assigned to two groups, intervention A and intervention B. Group A's menu was fortified with Pro-Cal™. Groups B's menu was fortified with the current fortifier (glucose polymer and skimmed milk powder). To minimize expectation bias and to optimise the validity of the research both the patient and the investigator were unaware of the treatment assigned making it a double blind clinical trial. The study was designed to fortify the patients lunch and dinner menu. Four foods were identified as having the most potential to be fortified, namely soup, mashed potato, custard and mousse.

Several outcomes were recorded from the intervention phase namely, biochemistry and dietary intakes. To monitor any effect of the supplement on blood biochemistry, venous blood samples were taken and measurements of haemoglobin, creatininie, urea, serum albumin, serum electrolytes and liver function were recorded on admission to the trail and on discharge.

The patient's diet was fortified for a period of 19.5 days average. During the period of fortification patients lunchtime and dinnertime dietary intakes were alternatively weighted on a daily basis and the nutritional content and level of fortification were analyzed using the nutritional analysis package Comp-eat. Standard portion weights and ingredients of all meals on the hospital menu have previously been entered onto the Comp-eat database. Therefore, by subtracting the plate (food residue left by patients) from the portion weight each patients' dietary intake could be assessed for nutritional content and the level of fortification could be calculated.

Additionally, a sensory evaluation of soup and custard having the composition Pro-Cal™ or the conventional fortifier (glucose polymer and skimmed milk) added thereto was carried out to assess the overall acceptability of these foods with the added fortifiers. Two samples of soup and two samples of custard each having one of the fortifiers added thereto were assessed in appearance, texture and flavour using the Hedonic scale.

## Results

Of the food consumed the mean energy intake increased by 55.1% (Intervention A) as opposed to 29.3% (Intervention B) (P = <0.0007) and protein increased by 32.39% (Invention A) as opposed to 14.93% (Intervention B) (P = < 0.0000).

Tables 10 and 11 below illustrate the percentage increase of energy of Groups A and B respectively. The mean energy intake pre-intervention of Group A was 572.8 kcal and the mean energy intake post intervention was 873.4 kcal, making a difference of +300.6kcal. The mean energy intake pre-intervention of Group B was 561 kcal and the mean energy intake post intervention was 719.6 kcal, a difference of +158.6 kcal.

Table 10

| Group A | | |
|---|---|---|
| **Patient No.** | **Energy (kcal)** | | **% Increase of** |
| | **Pre Intervention** | **Post Intervention** | **Energy** |
| 1 | 474 | 714 | 51% |
| 2 | 402 | 652 | 62% |
| 3 | 438 | 652 | 49% |
| 4 | 486 | 779 | 60% |
| 5 | 378 | 628 | 66% |
| 6 | 442 | 724 | 64% |
| 7 | 524 | 834 | 59% |
| 8 | 1028 | 1380 | 35% |
| 9 | 834 | 1284 | 54% |
| 10 | 722 | 1092 | 51% |

Table 10  (continued)

| Group A | | |
| --- | --- | --- |
| Patient No. | Energy (kcal) | | % Increase of |
| | Pre Intervention | Post Intervention | Energy |
| Mean | 572.8 | 873.9 | 55.1% |

Table 11

| Group B | | |
| --- | --- | --- |
| Patient No. | Energy (kcal) | | % Increase of |
| | Pre Intervention | Post Intervention | Energy |
| 11 | 502 | 739 | 47% |
| 12 | 392 | 521 | 33% |
| 13 | 998 | 1155 | 16% |
| 14 | 502 | 641 | 27% |
| 15 | 734 | 1085 | 47% |
| 16 | 362 | 471 | 30% |
| 17 | 418 | 521 | 25% |
| 18 | 806 | 931 | 16% |
| 19 | 474 | 595 | 26% |
| 20 | 422 | 530 | 26% |
| Mean | 561 | 719.6 | 29.3% |

Evidently there was no significant difference between the calorie intake pre-intervention between groups A & B. Therefore the higher increase in energy (55.1% as opposed to 29.3%, a significant difference of 25.8% (P = <0.0007)) appears to be achieved by the food fortification used in Intervention A.

With regard to the protein intakes, the mean protein intake pre intervention of Group A was 22.78g and the mean protein intake post intervention was 28.89g, a difference of +6.11 g. In contrast, the mean protein intake pre-intervention of Group B was 21.76g and the mean protein intake post intervention was 24.83g, a difference of +3.07g. Tables 12 and 13 below illustrate the percentage increase in protein intake for Groups A and B respectively.

Table 12

| Group A | | |
| --- | --- | --- |
| Patient No. | Protein (g) | | % Increase in Protein intake |
| | Pre Intervention | Post Intervention | |
| 1 | 23.28 | 28.14 | 20.87% |
| 2 | 18.88 | 23.88 | 26.48% |
| 3 | 18.50 | 22.83 | 23.40% |
| 4 | 22.30 | 28.23 | 26.60% |
| 5 | 13.50 | 18.56 | 37.48% |
| 6 | 16.84 | 22.55 | 33.91% |
| 7 | 23.00 | 29.27 | 27.26% |
| 8 | 36.38 | 43.67 | 20.03% |
| 9 | 30.26 | 39.38 | 30.13% |

Table 12 (continued)

| Group A | | | |
|---|---|---|---|
| Patient No. | Protein (g) | | % Increase in Protein intake |
| | Pre Intervention | Post Intervention | |
| 10 | 24.84 | 32.35 | 30.23% |
| Mean | 22.78 | 28.89 | 32.39% |

Table 13

| Group B | | | |
|---|---|---|---|
| Patient No. | Protein (g) | | % Increase in Protein intake |
| | Pre Intervention | Post Intervention | |
| 11 | 16.94 | 21.55 | 27% |
| 12 | 11.90 | 14.41 | 21% |
| 13 | 35.26 | 38.30 | 8.6% |
| 14 | 22.68 | 25.37 | 11.8% |
| 15 | 23.96 | 30.78 | 28% |
| 16 | 16.54 | 18.65 | 12.7% |
| 17 | 18.32 | 20.33 | 10.97% |
| 18 | 29.86 | 32.28 | 8.1% |
| 19 | 23.30 | 25.64 | 10% |
| 20 | 18.86 | 20.95 | 11.08% |
| Mean | 21.76 | 24.83 | 14.93% |

Again, there was no significant difference between the protein intake pre-intervention between groups A & B. Therefore the higher increase in protein (3 2.39%) as opposed to 14.93% (P = <0.000)) appears to be achieved by the food fortification used in intervention A.

As there was no significant difference (p= 0.13) between the food consumed excluding the fortifiers in the Group A & B this suggests that the obvious increase in calories and protein was due to the successful addition of Pro-Cal™.

The results of the Sensory Evaluation study in respect of soup and custard respectively is given in Tables 14 and 15 below:-

Table 14

| | Mean Values | | |
|---|---|---|---|
| Sample Code | Appearance | Texture | Flavour |
| 125 | 5.95 | 7.95 | 7.65 |
| 333 | 4.02 | 6.00 | 1.50 |

Table 15

| | Mean Values | | |
|---|---|---|---|
| Sample Code | Appearance | Texture | Flavour |
| 125 | 6.93 | 6.27 | 7.25 |
| 333 | 4.12 | 6.75 | 2.03 |

The Hedonic scale shown below was used to provide the description number which best described each sensory aspect of the coded sample.

| Hedonic Scale | | | |
|---|---|---|---|
| 8 - | like extremely | 7 - | like very much |
| 6 - | like moderately | 5 - | like slightly |
| 4 - | dislike slightly | 3 - | dislike moderately |
| 2 - | dislike very much | 1 - | dislike extremely |

The results clearly demonstrate that the composition of the present invention produces food of better quality than the conventional fortifier.

Weight gain is a poor indicator in renal patients due to changes in fluid status. However, in the trial there was no weight change in either Group A or B. Additionally, there was no change in mid-upper arm circumference in either Group A or Group B and typical blood results for a group of end stage renal failure patients on renal replacement therapy were obtained. No adverse events were reported during the trial.

The aim of this investigation was to compare the efficacy of a new food enricher (Pro-Cal™, Group A) to the current food fortification approach used at Manchester Royal Infirmary (glucose polymer and skimmed milk powder, Group B) in a group of patients with end stage renal failure pre dialysis or on renal replacement therapy who had a Nutritional Risk Score < 4. Increasing the energy and protein content by fortifying food is preferred to taking nutritional liquid supplements since renal patients should not significantly increase their fluid intake. The results of the study indicate that Pro-Cal™ is more effective than the conventional fortifier, with the mean calorie/protein intake being almost double that of the current approach.

There are several reasons why Pro-Cal™ may be so successful in improving the energy/protein intake of patients. Firstly, in a pre Sensory Evaluation Study to assess the appearance, texture, flavour and overall acceptability of food fortified with intervention A (Pro-Cal™) compared to food fortified with intervention B (glucose polymer and skimmed milk powder) intervention A, on average, scored higher on all counts. Additionally, due to the neutral taste and energy density of Pro-Cal™, a greater amount of calories and protein could be physically added to foods. In comparison, a glucose polymer is inherently sweet whilst skimmed milk powder is difficult to disperse to food and therefore both products are more difficult to disguise.

The addition of Pro-Cal™ is also a simplified method of fortifying food as only one product is required to provide both energy and protein, as opposed to using both a glucose polymer (kcal) and skimmed milk powder (protein).

Furthermore, generally CAPD patients are treated on an out patient capacity and Hemodialysis patents visit the hospital three times a week for treatment. Therefore it is particularly important to provide a nutritional regimen that will fit in with the patients life style at home. This approach of using food enrichers that are simple to use and can be added to foods familiar to the patient will ensurt better long term compliance.

The data suggests that Pro-Cal™ can be added at a greater level than alternative food fortifiers and can be added to a variety of food/drink, enriching the energy and protein content with the minimum effect of taste and texture. Therefore, it appears that Pro-Cal™ is more effective than current foods fortifiers.

Table 16 below compares the weights of current supplements with the weights of the compositions QuickCal™ and Pro-Cal™ for providing an energy intake of 100kcal. It is clear that a significantly smaller amount of the composition of the present invention is required to provide the same amount of energy to a patient.

Table 16

|  | Weight/Volume | Kcals | Protein |
|---|---|---|---|
| QuickCal | 13g | 100 | 0.6 |
| Pro-Cal | 15g | 100 | 2 |
| Glucose Polymer | 25g | 100 | 0 |
| Calogen | 22ml | 100 | 0 |
| Duocal | 20g | 100 | 0 |
| Ensure | 100ml | 100 | 4 |
| Ensure Plus | 67ml | 100 | 4 |

Table 15 below illustrates the amount of fat, carbohydrate and protein present in 100g of the two illustrated embodiments of the present invention, namely QuickCal™ and Pro-Cal™ compared to the amount present in other food fortifiers and supplements. The Table also identifies the form of the various compositions. It is clearly evident that the compositions of the present invention are the only compositions in powder form that provide a fat content in excess of 50% with a correspondingly high energy intake.

Table 17

|  | QuicCal 100g | Pro-Cal 100g | Calogen 100g | Duocal 100g | Scandishal 100g | Duobar 100g | Build Up 100g | Complan 100g |
|---|---|---|---|---|---|---|---|---|
| Fat | 77 | 56.2 | 50 | 22.3 | 24.2 | 49.8 | 0.7 | 14.8 |
| Carbohyd-Rate | 17 | 26.8 | 0 | 72.7 | 68.2 | 49.8 | 63 | 58.6 |
| Protein Max | 4.6 | 13.5 | 0 | 0 | 4.7 |  | 23.1 | 15.5 |
| kcals max | 779.4 | 667 | 450 | 491.5 | 514 | 647.4 | 350.7 | 429.6 |
| Form | powder | powder | liquid | Powder | Powder | solid | Powder | powder |

The use of food fortification has a great potential value in numerous hospital specialties and in the community. Food fortification is a simple and cost-effective way in which to prevent and treat malnutrition in the large and increasing number of elderly, malnourished patients. It appears that those patients with the greatest needs achieve the largest increase in energy intake. The key advantages of the compositions of the present invention are that, unlike using natural ingredients (e.g. cream), they can be disguised easily, do not add bulk to the food and only small amounts are wasted. As an approach to the prevention and treatment of malnutrition, food fortification using the compositions of the present invention is simple, effective and economical.

## Example 3

Another of the preferred compositions according to the present invention is a powder known as Vibre-Cal™ which is a blend of fat, protein, carbohydrate and fibre to provide a high energy food or drink enricher which also provides an additional fibre intake.
The typical composition of Vibre-Cal™ is given in Table 18 below:-

Table 18

|  | Per 100g | Per 12g |
|---|---|---|
| Energy kj (Kcal) | 2273.55 (543.70) | 272.83 (65.24) |
| Total Fibre (g) | 17.22 | 2.07 |
| Insoluble Fibre (g) | 8.27 | 0.99 |
| Soluble Fibre (g) | 8.96 | 1.07 |
| Protein equivalent (g) | 8.37 | 1.00 |
| Amino acids (g) | 0.00 | 0.00 |
| Carbohydrate (g) | 27.57 | 3.31 |
| Fat Total (g) | 43.44 | 5.21 |
| Saturated fatty acids (g) | 0.00 | 0.00 |
| Monounsaturated fatty acids (g) | 0.00 | 0.00 |
| Polyunsaturated fatty acids (g) | 0.00 | 0.00 |
| Moisture | 0.00 | 0.00 |
| MINERALS Sodium (mg) | 151.65 | 18.20 |
| Potassium (mg) | 291.76 | 35.01 |
| Calcium (mg) | 205.28 | 24.63 |

Table 18 (continued)

|  | Per 100g | Per 12g |
|---|---|---|
| Phosphorous (mg) | 164.57 | 19.75 |
| Magnesium (mg) | 20.90 | 2.51 |
| Chloride (mg) | 171.20 | 20.54 |
| Iron (mg) | 0.00 | 0.00 |
| Selenium (mcg) | 0.00 | 0.00 |

**Claims**

1.  A composition for fortifying foods and drinks **characterised in that** the composition is in powdered form and consists of nutrients in the following percentages by weight of the total composition:

    at least 40% by weight fat;
    15-30% by weight carbohydrate;
    3-30% by weight protein; and
    optionally, 10-25% by weight fibre and minor amounts of vitamins and/or minerals.

2.  A composition as claimed in claim 1 or 2 wherein the composition provides an energy intake of at least 5 kcal per gram.

3.  A composition for fortifying foods and drinks as claimed in claim 1 wherein the composition comprises nutrients in the following percentages by weight:

    | Fat | 77 |
    |---|---|
    | Carbohydrate | 17 |
    | Protein | 4.6 |

    to provide an energy intake of least 7 kcal per gram.

4.  A composition for fortifying foods and drinks as claimed in claim. 1 wherein the composition comprises nutrients in the following percentages by weight:-

    | Fat | 56.2 |
    |---|---|
    | Carbohydrate | 26.8 |
    | Protein | 13.5 |

    to provide an energy intake of at least 6 kcal per gram and a protein intake of at least 0.12g per gram.

5.  A composition as claimed in claim 1 wherein 15 to 20% of the composition is fibre.

6.  A composition for fortifying foods and drinks as claimed in claim 5 wherein the composition comprises nutrients in the following percentages by weight:

    | Fat | 43.4 |
    |---|---|
    | Carbohydrate | 27.6 |
    | Protein | 8.4 |
    | Fibre | 17.2 |

    to provide an energy intake of at least 5 kcal per gram, a protein intake of at least 0.075g per gram and a fibre intake of at least 0.15g per gram.

7.  A food or drink fortifier comprising a composition as claimed in any one of the preceding claims.

8. The use of a composition for fortifying food or drink as claimed in any one of the preceding claims for the preparation of a composition for the treatment and/or prevention of malnutrition.

9. A method for fortifying a food or drink comprising the steps of adding to a food or drink a powdered composition consisting of at least 40% by weight fat, 15-30% by weight carbohydrate, 3-30% by weight protein and optionally, 10-25% by weight fibre and minor amounts of vitamins and/or minerals.

10. A method as claimed in claim 9 wherein the composition is provided in a sachet holding a specified weight to provide a predetermined amount of energy from the sachet.

11. A method as claimed in claim 9 wherein the component is measured in a scoop for adding to the food or drink.

**Patentansprüche**

1. Zusammensetzung zur Nahrungsergänzung und von Getränken, **dadurch gekennzeichnet, dass** sie in Pulverform vorliegt und aus Nahrungsstoffen in den folgenden Gewichtsprozenten der gesamten Zusammensetzung besteht:

   wenigstens 40 Gew-% Fett;
   15 bis 30 Gew.-% Kohlenhydrat;
   3 bis 30 Gew.-% Protein; und
   gegebenenfalls 10 bis 25 Gew.-% Fasern und geringe Anteile von Vitaminen und/oder Mineralien.

2. Zusammensetzung gemäß Anspruch 1 oder 2, bei der die Zusammensetzung eine Energieaufnahzne von wenigstens 5 kcal. pro Gramm zur Verfügung stellt.

3. Zusammensetzung zur Nahrungsergänzung und von Getränken gemäß Anspruch 1, bei der die Zusammensetzung Nahrungsstoffe in den folgenden Gewichtsprozenten umfasst:

| Fett | 77 |
|---|---|
| Kohlenhydrat | 17 |
| Protein | 4,6 |

um eine Energieaufnahme von wenigstens 7 kcal. pro Gramm zur Verfügung zu stellen.

4. Zusammensetzung zur Nahrungsergänzung und von Getränken gemäß Anspruch 1, bei der die Zusammensetzung Nahrungsstoffe in den folgenden Gewichtsprozenten aufweist:

| Fett | 56,2 |
|---|---|
| Kohlenhydrat | 26,8 |
| Protein | 13,5 |

um eine Energieaufnahme von mindesten 6 kcal. pro Gramm und eine Proteinaufnahme von wenigstens 0,12g pro Gramm zur Verfügung zu stellen.

5. Zusammensetzung gemäß Anspruch 1, bei der 15 bis 20 % der Zusammensetzung Fasern sind.

6. Zusammensetzung zur Nahrungsergänzung und von Getränken gemäß Anspruch 5, bei der die Zusammensetzung Nahrungsstoffe in den folgenden Gewichtsprozenten umfasst:

| Fett | 43,4 |
|---|---|
| Kohlenhydrate | 27,6 |
| Protein | 8,4 |
| Fasern | 17,2 |

um eine Energieaufnahme von 5 kcal. pro Gramm, eine Proteinaufnahme von wenigstens 0,075 pro Gramm und eine Faseraufnahme von wenigstens 0,15g pro Gramm zur Verfugung zu stellen.

7. Nahrungsmittel- oder Getränkeergänzungsmittel, umfassend eine Zusammensetzung gemäß irgendeinem der vorhergehenden Ansprüche.

8. Verwendung einer Zusammensetzung zur Nahrungsergänzung oder von Getränken gemäß irgendeinem der vorhergehenden Ansprüche zur Herstellung einer Zusammensetzung zur Behandlung und/oder Prävention von Unterernährung.

9. Verfahren zur Ergänzung eines Nahrungsmittels oder Getränks, umfassend die Schritte der Zugabe zu einem Getränk oder eines Nahrungsmittels einer pulverförmigen Zusammensetzung, bestehend aus wenigstens 40 Gew.-% Fett, 15 bis 30 Gew.-% Kohlenhydraten, 3 bis 30 Gew.-% Protein und gegebenenfalls 1.0 bis 25 Gew.-% Fasern und geringen Anteilen an Vitaminen und/oder Mineralien.

10. Verfahren gemäß Anspruch 9, bei dem die Zusammensetzung in einer Portionspackung zur Verfügung gestellt wird, die eine spezifizierte Masse aufnimmt, um eine vorbestimmte Energiemenge aus der Portionspackung zur Verfügung zu stellen.

11. Verfahren gemäß Anspruch 9, bei dem die Komponente in einem Behälter zur Zugabe zu dem Nahrungsmittel oder Getränk gemessen wird.

**Revendications**

1. Composition pour fortifier de la nourriture et de la boisson **caractérisées en ce que** la composition se présente sous forme de poudre et consiste en des nutriments dans les pourcentages massiques suivants de la composition totale :

    ➢ au moins 40 % en masse de graisse,
    ➢ 15 à 30 % en masse de glucides,
    ➢ 3 à 30 % en masse de protéines, et
    ➢ facultativement, 10 à 25 % en masse de fibres et de faibles quantités de vitamines et / ou de minéraux.

2. Composition selon la revendication 1 dans laquelle la composition fournit un apport d'énergie d'au moins 5 kcal par gramme.

3. Composition pour fortifier de la nourriture et de la boisson selon la revendication 1 dans laquelle la composition comprend des nutriments dans les pourcentages massiques suivants :

    ➢ Graisse 77
    ➢ Glucides 17
    ➢ Protéines 4,6

pour fournir un apport d'énergie d'au moins 7 kcal par gramme.

4. Composition pour fortifier de la nourriture et de la boisson selon la revendication 1 dans laquelle la composition comprend des nutriments dans les pourcentages massiques suivants :

    ➢ Graisse 56,2
    ➢ Glucides 26,8
    ➢ Protéines 13,5

Pour fournir un apport d'énergie d'au moins 6 kcal par gramme et un apport de protéines d'au moins 0, 12 g par gramme.

5. Composition selon la revendication 1 dans laquelle 15 à 20 % de la composition sont des fibres.

**6.** Composition pour fortifier de la nourriture et de la boisson selon la revendication 5 dans laquelle la composition comprend des nutriments dans les pourcentages massiques suivants ;

> ➢ Graisse 43,4
> ➢ Glucides 27,6
> ➢ Protéines 8,4
> ➢ Fibres 17,2

Pour fournir un apport d'énergie d'au moins 5 kcal par gramme, un apport de protéines d'au moins 0,075 g par gramme et un apport de fibres d'au moins 0,15 g par gramme.

**7.** Fortifiant pour nourriture ou pour boisson comprenant une composition selon l'une quelconque des revendications précédentes.

**8.** Utilisation d'une composition pour fortifier de la nourriture ou de la boisson selon l'une quelconque des revendications précédentes pour la préparation d'une composition pour le traitement et / ou la prévention, de la malnutrition.

**9.** Méthode pour fortifier de la nourriture ou de la boisson comprenant les étapes d'ajouter à de la nourriture ou de la boisson une composition se présentant sous forme de poudre consistant en au moins 40% en masse de graisse, 15 à 30 % en masse de glucides, 3 à 30 % en masse de protéines et facultativement, 10 à 25 % en masse de fibres et de faibles quantités de vitamines et / ou de minéraux.

**10.** Méthode selon la revendication 9 dans laquelle la composition est présentée dans un sachet contenant un poids spécifique afin de fournir un apport prédéterminé d'énergie à partir du sachet.

**11.** Méthode selon la revendication 9 dans laquelle le composant est mesuré dans une pelle afin de l'ajouter à la nourriture ou la boisson.

FIG.1

FIG.2

## FIG.3

## FIG.4

## FIG.5